# EUROPÄISCHE PATENTSCHRIFT

(11) **EP 2 016 979 B1**
(45) Veröffentlichungstag und Bekanntmachung des Hinweises auf die Patenterteilung: **08.06.2011**
(21) Anmeldenummer: 08104473.7
(22) Anmeldetag: 19.06.2008
(51) Int. Cl.: A61N 5/10

(54) **Partikelstrahlapplikationsvorrichtung, Bestrahlungsvorrichtung sowie Verfahren zur Führung eines Partikelstrahls**
Particle beam application device, beam emitting device and method for guiding a particle beam
Dispositif d'application par rayon de particules, dispositif de rayonnement et procédé de guidage d'un rayon de particules

(30) Priorität: 20.07.2007 DE 102007033894; 20.07.2007 US 961348 P
(43) Veröffentlichungstag der Anmeldung: 21.01.2009
(73) Patentinhaber: SIEMENS AKTIENGESELLSCHAFT, 80333 München (DE)
(72) Erfinder: Hansmann, Thomas, 69117 Heidelberg (DE); Rietzel, Eike, 64289 Darmstadt (DE)

(56) Entgegenhaltungen:
- EP-A- 1 482 519
- JP-A- 2000 214 298
- US-A- 5 668 371

## Beschreibung

Die Erfindung betrifft eine Partikelstrahlapplikationsvorrichtung, wie sie insbesondere im Rahmen der Partikeltherapie zur Bestrahlung von Gewebe mit einem Partikelstrahl eingesetzt wird. Weiterhin betrifft die Erfindung eine Bestrahlungsvorrichtung mit einer derartigen Partikelstrahlapplikationsvorrichtung. Weiterhin betrifft die Erfindung ein Verfahren zur Führung eines Partikelstrahls.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, können jedoch auch in nicht-therapeutischen Gebieten Anwendung finden kann. Hierzu gehören beispielsweise Forschungsarbeiten im Rahmen der Partikeltherapie, die an nicht-lebenden Phantomen oder Körpern durchgeführt werden, Bestrahlungen von Materialien, etc. Hierbei werden üblicherweise geladene Partikel auf hohe Energien beschleunigt, zu einem Partikelstrahl geformt und auf ein zu bestrahlendes Objekt gerichtet. Der Partikelstrahl dringt in das Objekt ein und gibt dort an einem definierten Ort seine Energie ab, was zu einer Zerstörung des sich dort befindlichen Gewebes führt. Als Partikel kommen üblicherweise Protonen, Kohlenstoffionen aber auch Pionen, Heliumionen oder andere Ionensorten zum Einsatz.

Nachdem Partikel beschleunigt sind und ein Partikelstrahl geformt ist, besteht die Notwendigkeit, den Partikelstrahl möglichst zielgenau auf ein zu bestrahlendes Zielvolumen zu richten, damit die Energie des Partikelstrahls möglichst genau an einem vorgegebenen Zielort deponiert wird. Hierzu existieren verschiedene bekannte Verfahren.

Eine Möglichkeit ist es, den Partikelstrahl flächig aufzuweiten, so dass sein Querschnitt so groß wird, dass große Teile des zu bestrahlenden Zielvolumens - oder sogar das gesamte Zielvolumen - von dem aufgeweiteten Partikelstrahl getroffen werden. Ein solches Verfahren ist auch unter dem Namen "Scattering-Verfahren" bekannt. In diesem Zusammenhang werden oftmals Kollimatoren eingesetzt, die in den Strahlengang des aufgeweiteten Partikelstrahls eingebracht werden, und mit denen die Querschnittsfläche des Partikelstrahls geformt wird. Hierdurch lässt sich beispielsweise der Querschnitt des Partikelstrahls an ein zu bestrahlendes Zielvolumen anpassen. Eine Tiefenmodulation, d.h. eine Eindringtiefe des Partikelstrahls, kann bei dem Scattering-Verfahren erreicht werden, indem die Energie des Partikelstrahls variiert wird.

Eine andere bekannte Möglichkeit ist das so genannte Rasterscan-Verfahren, bei der ein Partikelstrahl mit einer vergleichsweise geringen Querschnittsfläche von einigen wenigen Millimetern (so genannter "pencil beam") auf das Zielvolumen gelenkt wird. Durch Ablenken des geladenen Partikelstrahls können nacheinander verschiedene Rasterpunkte im Zielvolumen angefahren werden, so dass der Partikelstrahl seine Energie sequenziell an einzelnen Rasterpunkten im Zielvolumen abgibt. Eine Eindringtiefe des Partikelstrahls in das Zielvolumen und damit auch die Ansteuerung von Rasterpunkten, die in unterschiedlicher Tiefe im Zielvolumen liegen, wird über eine Variation der Energie des Partikelstrahls erreicht.

Das US Patent 5,668,371 (Deasy et al.) zeigt eine Partikeltherapieanlage, bei der ein fächerförmiger Strahl mit einem Magnetfeldgeneriert wird und ein Patient zur Bestrahlung relativ zum mit einem Magnetfeld fächerförmigen Strahl rotiert wird.

Es ist die Aufgabe der Erfindung, eine Partikelstrahlapplikationsvorrichtung anzugeben, die eine Applikation eines Partikelstrahls mit geringer Streustrahlung und guter Unempfindlichkeit gegenüber einer Bewegung des Zielvolumens bei gleichzeitig schneller Bestrahlung des Zielvolumens ermöglicht. Weiterhin ist es die Aufgabe der Erfindung eine Bestrahlungsvorrichtung mit einer derartigen Partikelstrahlapplikationsvorrichtung anzugeben. Weiterhin ist es die Aufgabe der Erfindung ein Verfahren zur Führung eines Partikelstrahls anzugeben, mit dem es ermöglicht wird, einen Partikelstrahl bereitzustellen, mit dem eine schnelle Bestrahlung eines Zielvolumens bei geringer Belastung durch Streustrahlung ermöglicht wird.

Die Aufgabe der Erfindung wird demnach gelöst durch die Gegenstände der unabhängigen Ansprüche.

Mit der erfindungsgemäßen Partikelstrahlapplikationsvorrichtung kann ein Partikelstrahl, der in die Partikelstrahlapplikationsvorrichtung eintritt, auf ein Zielvolumen zur Bestrahlung des Zielvolumens gerichtet werden. Hierzu weist die Partikelstrahlapplikationsvorrichtung eine im Strahlengang des Partikelstrahls anordenbare Strahlformungsvorrichtung zur Formung eines Querschnittsprofils des Partikelstrahls auf. Die Strahlformungsvorrichtung ist dabei derart ausgebildet, dass der Partikelstrahl nach Formung durch die Strahlformungsvorrichtung ein linienförmiges Querschnittsprofil aufweist.

Unter einem linienförmigen Querschnittsprofil wird dabei ein Querschnittsprofil verstanden, das in einer Richtung eine wesentlich größere Ausdehnung hat als in einer anderen Richtung, so dass das Querschnittsprofil insgesamt linienförmig erscheint. Die Ausdehnung in einer Richtung entspricht dabei der Ausdehnung eines Partikelstrahls, wie er beispielsweise im Rasterscan-Verfahren eingesetzt wird. Beispielsweise kann die Ausdehnung in einer Richtung wenige Millimeter betragen, während die Ausdehnung in der anderen Richtung im Zentimeterbereich liegt. Hierdurch unterscheidet sich das Querschnittsprofil des Partikelstrahls von bekannten Querschnittsprofilen. Beim Rasterscan-Verfahren ist das Querschnittsprofil des Partikelstrahls punktförmig, mit einer Ausdehnung des Partikelstrahls von z.B. wenigen Millimetern in jeder Richtung, während bei einem flächig aufgeweiteten Partikelstrahl der Querschnitt des Partikelstrahls eine deutliche Ausdehnung in allen zwei Dimensionen hat, wobei die genaue Form der flächigen Querschnittsfläche oftmals durch einen begrenzenden Kollimator geformt wird.

Zusätzlich umfasst die Partikelstrahlapplikationsvorrichtung eine Steuervorrichtung zum Ändern der Relativposition des Partikelstrahls und des Zielvolumens während eines Bestrahlungsvorgangs. Hierbei ist die Steuervorrichtung derart ausgebildet, dass durch eine Änderung der Relativposition der Partikelstrahl mit dem linienförmigen Querschnittsprofil über das Zielvolumen gescannt werden kann.

Der Erfindung liegt folglich die Idee zu Grunde, dass herkömmliche Bestrahlungsverfahren wie beispielsweise das Rasterscan-Verfahren oder das Scattering-Verfahren mit einem flächig aufgeweiteten Partikelstrahl mit Nachteilen einhergehen. Das flächige Aufweiten des Partikelstrahls und das anschließende Begrenzen des Partikelstrahls mit einem Kollimator erzeugt meistens eine erhebliche Streustrahlung, die die Genauigkeit der Bestrahlung eines Zielvolumens herabsetzt. Andererseits ist das Rasterscan-Verfahren anfällig für Über- oder Unterdosierungen, falls sich das Zielvolumen bewegt, was bei der Bestrahlung von menschlichem Gewebe oftmals vorkommt.

Mit der Partikelstrahlapplikationsvorrichtung kann nun ein Partikelstrahl erzeugt werden, der im Gegensatz zu einem flächigen oder punktförmigem Querschnittsprofil ein linienförmiges Querschnittsprofil aufweist. Hierbei wird erheblich weniger Streustrahlung erzeugt als bei einer flächigen Aufweitung des Partikelstrahls. Mit der Steuervorrichtung zum Ändern der Relativposition zwischen dem Partikelstrahl und dem Zielvolumen ist es möglich, den Partikelstrahl mit dem linienförmigen Querschnittsprofil über das Zielvolumen zu scannen. Durch das linienförmige Querschnittsprofil ist das Scan-Verfahren weniger anfällig für Über- oder Unterdosierungen bei der Bestrahlung eines bewegten Zielvolumens.

Die Strahlformungsvorrichtung kann dabei von einer Steuervorrichtung für die Strahlformungsvorrichtung gesteuert werden, die derart ausgebildet ist, dass die Strahlformungsvorrichtung mit dem Partikelstrahl derart zusammenwirkt, dass ein aus der Strahlformungsvorrichtung austretender Partikelstrahl ein linienförmiges Querschnittsprofil aufweist. Die Steuervorrichtung zum Ändern der Relativposition kann dabei insbesondere in Strahlrichtung hinter der Strahlformungsvorrichtung angeordnet werden.

Erfindungsgemäß umfasst die Strahlformungsvorrichtung ein Magnetsystem, mit dem ein oszillierendes Magnetfeld erzeugt werden kann. Ein Partikelstrahl mit einem punktförmigen Querschnittsprofil, der durch dieses oszillierende Magnetfeld gelenkt wird, wird derart hin- und hergelenkt, dass durch die Oszillation des Partikelstrahls mit dem punktförmigen Querschnittsprofil ein Partikelstrahl mit einem linienförmigen Querschnittsprofil geformt wird. Das oszillierende Magnetfeld ist dabei relativ zum Partikelstrahl derart orientiert, dass eine Ablenkung des Partikelstrahls in eine Richtung erfolgt, die im Wesentlichen senkrecht zur Strahlverlaufsrichtung verläuft. Diese Ausführungsform hat den Vorteil, dass eine Partikelstrahlapplikationsvorrichtung, mit der ein Rasterscan-Verfahren durchgeführt werden kann, nur geringfügig modifiziert werden muss, um ein linienförmiges Querschnittsprofil im Partikelstrahl zu erzeugen.

Erfindungsgemäß wird das Magnetsystem durch eine Magnetsystemsteuervorrichtung gesteuert, mit der ein Offset und/oder eine Amplitude und/oder eine Frequenz des oszillierenden Magnetfeldes eingestellt werden kann. Hierdurch ist es möglich, das Profil des linienförmigen Querschnittsprofils mit einfachen Mitteln zu beeinflussen und zu formen. Insbesondere kann das oszillierende Magnetfeld während eines Bestrahlungsvorgangs variabel eingestellt werden. Hierdurch ist es möglich, den linienförmigen Partikelstrahl zu verändern, während er über ein Zielvolumen gescannt wird. Auf diese Weise kann der Partikelstrahl genau an das Zielvolumen angepasst werden.

Es ist weiterhin offenbart, dass die Strahlformungsvorrichtung einen Kollimator mit einer linienförmigen Öffnung umfassen kann, der in den Strahlengang des Partikelstrahls einbringbar ist. Insbesondere umfasst die Strahlformungsvorrichtung eine Streuvorrichtung zur Aufweitung des Partikelstrahls, die in Strahlrichtung vor dem Kollimator angeordnet ist. Mit dieser einfachen Strahlformungsvorrichtung kann aus einem insbesondere flächig aufgeweiteten Partikelstrahl mit einfachen Mitteln ein Partikelstrahl mit einem linienförmigen Querschnittsprofil erzeugt werden.

Es ist weiterhin offenbart, dass der Kollimator eine Einstellvorrichtung zum Ändern der Geometrie der linienförmigen Öffnung des Kollimators umfassen kann. Beispielsweise kann die Länge der linienförmigen Öffnung variabel eingestellt werden, d.h., dass die Länge der linienförmigen Öffnung begrenzt werden kann. Auf diese Weise kann das Querschnittsprofil des Partikelstrahls auf einfache Weise geändert werden. Es ist aber auch möglich, die linienförmige Öffnung in ihrem Längsverlauf mit der Einstellvorrichtung zu unterbrechen, so dass mit dem Partikelstrahl, der dann ein unterbrochenes linienförmiges Querschnittsprofil aufweist, auch toroidal geformte Zielvolumina bestrahlt werden können. Die Änderung der Geometrie der linienförmigen Öffnung bzw. des linienförmigen Querschnittsprofils kann dabei insbesondere während eines Bestrahlungsvorgangs und während des Scannens des Partikelstrahls über das Zielvolumen erfolgen.

Es ist weiterhin offenbart, dass der Kollimator derart ausgebildet sein kann, dass eine Position der linienförmigen Öffnung relativ zum Strahlverlauf, insbesondere senkrecht zum Strahlverlauf, verändert werden kann. Diese Veränderung kann durch die Steuervorrichtung zum Ändern der Relativposition zwischen dem Partikelstrahl und dem zu bestrahlenden Zielvolumen gesteuert werden. Hierdurch ist der Partikelstrahl mit dem linienförmigen Querschnittsprofil auf einfache Weise durch Bewegen der linienförmigen Öffnung im Strahlengang über das Zielvolumen scanbar. Dies kann beispielsweise dadurch erreicht werden, dass der Kollimator mit der linienförmigen Öffnung insgesamt relativ zum Strahlverlauf bewegt wird. Es kann aber auch dadurch erreicht werden, dass der Kollimator räumlich feststeht und der Kollimator derart ausgebildet ist, dass die linienförmige Öffnung im Kollimator bewegt wird.

In einer vorteilhaften Ausführungsform weist die Partikelstrahlapplikationsvorrichtung eine elektromagnetische Ablenkvorrichtung auf, die insbesondere im Strahlverlauf hinter der Strahlformungsvorrichtung angeordnet ist. Die elektromagnetische Ablenkvorrichtung kann den Partikelstrahl, der durch die elektromagnetische Ablenkvorrichtung tritt, ablenken und damit über das zu bestrahlende Zielvolumen scannen. Die elektromagnetische Ablenkvorrichtung wird dabei insbesondere durch die Steuervorrichtung zum Ändern der Relativposition zwischen dem Partikelstrahl und dem zu bestrahlenden Zielvolumen gesteuert. Eine derartige Ausführungsform weist den Vorteil auf, dass eine bekannte Vorrichtung zum Ablenken des Partikelstrahls, mit der ein Rasterscan-Verfahren durchgeführt werden kann, nur geringfügig modifiziert werden muss.

In einer anderen Ausführungsvariante ist die Steuervorrichtung zum Ändern der Relativposition zwischen dem Partikelstrahl und dem zu bestrahlenden Zielvolumen zur Abgabe eines Steuersignals für eine Positioniervorrichtung für das Zielvolumen ausgebildet, so dass während eines Bestrahlungsvorgangs das Zielvolumen mit der Positioniervorrichtung derart bewegt werden kann, dass der Partikelstrahl mit dem linienförmigen Querschnittsprofil über das Zielvolumen gescannt wird. Diese Ausführungsvariante hat den Vorteil, dass ein Scannen des Partikelstrahls über das Zielvolumen auf einfache Weise erreicht werden kann, ohne den Partikelstrahl selbst von einer gedachten Strahlmittelachse auszulenken. Die Partikelstrahlapplikationsvorrichtung kann dabei insbesondere die Positioniervorrichtung für das Zielvolumen mit umfassen.

Die Partikelstrahlapplikationsvorrichtung kann zusätzlich zumindest einen im Strahlverlauf hinter der Strahlformungsvorrichtung angeordneten Dipolmagneten zur Reinigung des Partikelstrahls von Streustrahlung aufweisen. Diese Ausführungsvariante hat den Vorteil, dass insbesondere bei einer eingesetzten Streuvorrichtung zur Aufweitung des Partikelstrahls und/oder einem Kollimator, der im Strahlengang des Partikelstrahls angeordnet wird, die Strahlqualität verbessert wird. In diesen Fällen wird nicht zu vernachlässigende Streustrahlung erzeugt, die dadurch charakterisiert ist, dass deren Masse-zu-Ladungs-Verhältnis und/oder deren Energie von den jeweils gewünschten Eigenschaften des Partikelstrahls abweicht. Wenn nun der Partikelstrahl durch den Dipolmagneten hindurch geschickt wird, werden die Komponenten des Partikelstrahls, die eine unterschiedliche Energie bzw. ein unterschiedliches Masse-zu-Ladungs-Verhältnis aufweisen, im Dipolmagneten anders abgelenkt als die Komponenten des Partikelstrahls, die die gewünschte Energie bzw. das gewünschte Masse-zu-Ladungs-Verhältnis aufweisen. Hierdurch kann der Partikelstrahl von ungewünschten Komponenten gereinigt werden, wodurch sich die Qualität einer Bestrahlung erhöht.

Insbesondere kann die Partikelstrahlapplikationsvorrichtung einen im Strahlverlauf hinter den Dipolmagneten angeordneten zweiten Kollimator mit einer linienförmigen Öffnung aufweisen. Mit einem derartigen zweiten Kollimator können die gewünschten Komponenten des Partikelstrahls auf einfache Weise ausgefiltert werden, da sie durch die unterschiedliche Ablenkung in Dipolmagneten nicht mehr durch die linienförmige Öffnung des zweiten Kollimators treffen.

In einer vorteilhaften Ausführungsvariante ist der zweite Kollimator derart ausgebildet, dass eine Position der linienförmigen Öffnung des zweiten Kollimators relativ zum Strahlverlauf veränderbar ist. Auf diese Weise kann der zweite Kollimator bzw. die linienförmige Öffnung des zweiten Kollimators an der Stelle positioniert werden, an der der Partikelstrahl mit dem linienförmigen Querschnittsprofil durch den zweiten Kollimator treffen soll, d.h. an der Sollposition des Partikelstrahls.

Insbesondere, wenn die Steuervorrichtung zum Ändern der Relativposition zwischen dem Partikelstrahl und dem zu bestrahlenden Zielvolumen die Position des Partikelstrahls ändert, kann so die Position der linienförmigen Öffnung des zweiten Kollimators in entsprechender Weise positioniert werden, um einen Durchtritt des Partikelstrahls durch den zweiten Kollimator mit jedem Strahlverlauf des Partikelstrahls zu ermöglichen.

Die erfindungsgemäße Bestrahlungsvorrichtung umfasst:
- zumindest eine Partikelstrahlapplikationsvorrichtung nach einem der Ansprüche 1 bis 13,
- zumindest eine Quelle zur Erzeugung von Partikeln
- zumindest eine vor der Partikelstrahlapplikationsvorrichtung angeordnete Beschleunigungsvorrichtung zur Beschleunigung der Partikel und zur Erzeugung des Partikelstrahls aus den beschleunigten Partikeln.

Hierdurch ist es möglich, ein Gesamtsystem zum Bestrahlen eines zu bestrahlenden Zielvolumens bereitzustellen, mit dem sowohl der Partikelstrahl erzeugt und bereitgestellt werden kann, mit dem aber auch der Partikelstrahl wie beschrieben geformt und auf das Zielvolumen gelenkt werden kann.

Eine derartige Bestrahlungsvorrichtung umfasst üblicherweise auch eine Positioniervorrichtung für das Zielvolumen.

Bei einer derartigen Bestrahlungsvorrichtung ist eine Relativposition eines bewegten Zielvolumens und dem Partikelstrahl insbesondere derart einstellbar, dass eine Hauptbewegungsrichtung des Zielvolumens parallel zum linienförmigen Querschnittsprofil des Partikelstrahls verläuft. Dies kann beispielsweise durch ein entsprechendes Positionieren des Zielvolumens mit der Positioniervorrichtung auf einfache Weise erreicht werden. Hierdurch ergibt sich eine besonders geringe Anfälligkeit für Über- bzw. Unterdosierungen bei der Bestrahlung eines bewegten Zielvolumens.

Die Partikelstrahlapplikationsvorrichtung kann insbesondere weitere Elemente zur Formung der Strahlqualität bzw. der Strahleigenschaften umfassen, wie z.B. eine Tiefenmodulationsvorrichtung zur Beeinflussung der Partikelstrahlenergie. Mit einer derartigen Tiefenmodulationsvorrichtung kann die Eindringtiefe des Partikelstrahls in das Zielvolumen moduliert werden. Zur Beeinflussung der Partikelstrahlenergie können beispielsweise ein so genanntes Modulator-Wheel oder ein so genannter Ridge-Filter eingesetzt werden.

Das erfindungsgemäße Verfahren zur Führung eines Partikelstrahls weist folgende Schritte auf:
- Formen eines Querschnittsprofils eines Partikelstrahls derart, dass der Partikelstrahl ein linienförmiges Querschnittsprofil aufweist,
- Ändern eines Verlaufs des Partikelstrahls in einer Richtung, die senkrecht zur Strahlverlaufsrichtung des Partikelstrahls und insbesondere senkrecht zum linienförmigen Verlauf des linienförmigen Querschnittsprofils verläuft.

Die Änderung des Verlaufs des Partikelstrahls kann insbesondere variabel erfolgen.

Erfindungsgemäß wird bei dem Formen des Querschnittsprofils des Partikelstrahls ein oszillierendes Magnetfeld auf den Partikelstrahl, der ein punktförmiges Querschnittsprofil aufweist, eingestrahlt. Hierdurch wird der Partikelstrahl in einer Richtung, die im Wesentlichen senkrecht zur Strahlverlaufsrichtung verläuft, oszillierend abgelenkt. Dadurch wird aus einem Partikelstrahl mit einem punktförmigen Querschnittsprofil ein Partikelstrahl mit einem linienförmigen Querschnittsprofil erzeugt. Das oszillierende Magnetfeld ist in seinem Offset, in seiner Amplitude und/oder in seiner Frequenz variabel einstellbar.

Es ist weiterhin offenbart, dass das Querschnittsprofil des Partikelstrahls dadurch geformt wird, dass der Partikelstrahl durch einen Kollimator mit einer linienförmigen Öffnung geführt wird. Insbesondere wird der Partikelstrahl zuvor kegelförmig aufgeweitet.

Es ist weiterhin offenbart, dass das Ändern des Verlaufs des Partikelstrahls dann dadurch erfolgen kann, dass die Position der linienförmigen Öffnung des Kollimators relativ zum Strahlverlauf, insbesondere senkrecht zum Strahlverlauf geändert wird.

In einer anderen Ausführungsform kann das Ändern des Verlaufs des Partikelstrahls dadurch erfolgen, dass sein Magnetfeld senkrecht zu Verlaufsrichtung des Partikelstrahls eingestrahlt wird, wodurch sich der Verlauf des Partikelstrahls ändert. Durch Variieren des Magnetfelds wird der Verlauf des Partikelstrahls variabel abgeändert, so dass beispielsweise ein Scan-Vorgang ermöglicht wird.

Nach Formen und Ändern des Verlaufs des Partikelstrahls kann der Partikelstrahl von Streustrahlung gereinigt werden, indem der Partikelstrahl durch ein weiteres, zweites Magnetfeld geführt und in seiner Richtung abgelenkt wird. Insbesondere kann der Partikelstrahl nach der Reinigung von Streustrahlung ein weiteres Mal in seinem Querschnittsprofil insbesondere durch einen Kollimator geformt werden.

Ausführungsformen der vorliegenden Erfindung mit vorteilhaften Weiterbildungen gemäß den Merkmalen der Unteransprüche werden nun anhand der folgenden Zeichnung näher erläutert, ohne jedoch darauf beschränkt zu sein. Es zeigen:
- Fig. 1: einen schematischen Überblick über den Aufbau einer Partikeltherapieanlage,
- Fig. 2: einen schematischen Überblick über den Aufbau einer Partikelstrahlapplikationsvorrichtung,
- Fig. 3: einen schematischen Überblick über eine Partikel- strahlapplikationsvorrichtung mit einem Kollimator mit einer linienförmigen Öffnung,
- Fig. 4: einen schematischen Überblick über eine Partikel- strahlapplikationsvorrichtung, mit der ein Scannen ei- nes Partikelstrahls mit einem linienförmigen Quer- schnittsprofil durch Bewegen des Zielvolumens erfolgt,
- Fig. 5: einen schematischen Überblick über eine erfindungsge- mäße Partikelstrahlapplikationsvorrichtung mit einem Magnetsystem und einer elektromagnetischen Ablenkvor- richtung zur Formung und Ablenkung des Partikel- strahls,
- Fig. 6: eine Draufsicht auf eine Partikelstrahlapplikations- vorrichtung, bei der der geformte Partikelstrahl mit- hilfe eines Dipolmagneten gereinigt wird, und
- Fig. 7: einen schematischen Überblick über einzelne Verfah- rensschritte.

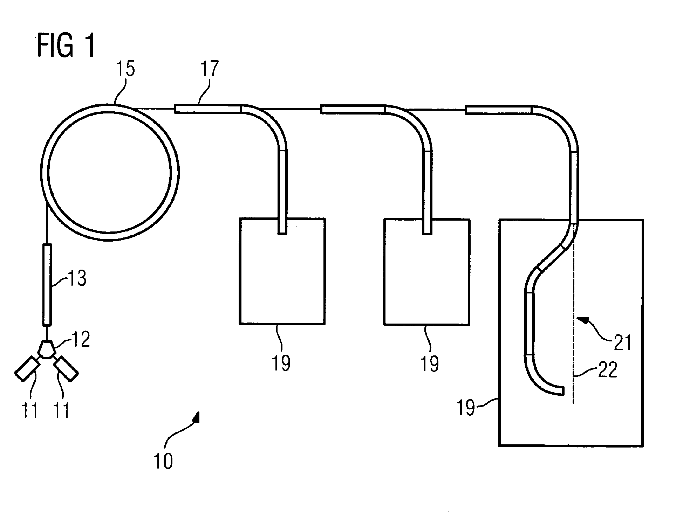

Figur 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Grundaufbau einer Partikeltherapieanlage 10 ist typisch für viele Partikeltherapieanlagen, kann aber auch hiervon abweichen; beispielsweise muss, je nach Beschleunigung der Partikel, eine Bestrahlungsvorrichtung nicht als Partikeltherapieanlage angeordnet sein.

Die nachfolgend beschriebenen Ausführungsbeispiele sind sowohl in Zusammenhang mit der anhand von Fig. 1 dargestellten Partikeltherapieanlage als auch mit anderen Partikeltherapieanlagen oder Strahlentherapieanlagen einsetzbar.

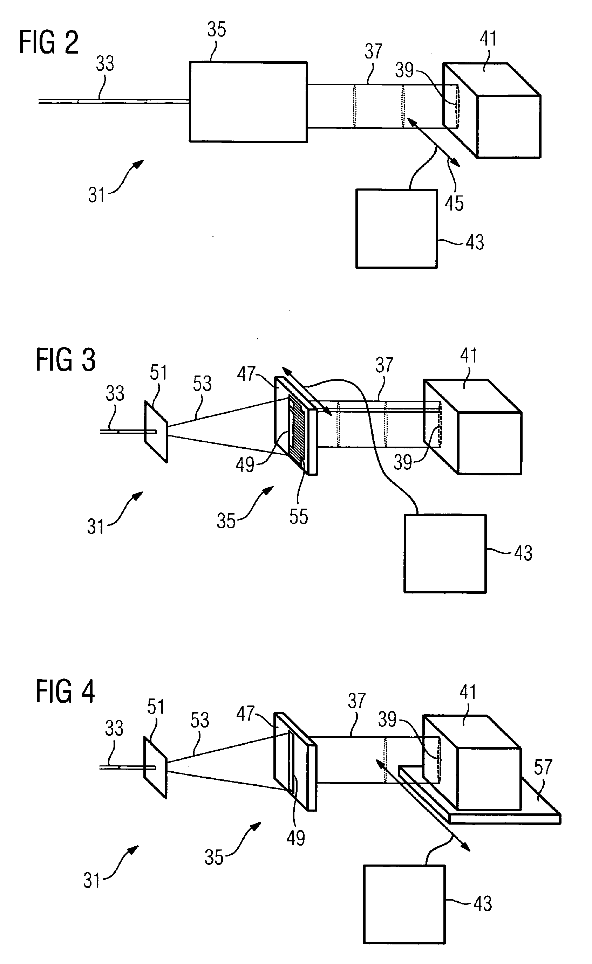

Fig. 2 zeigt schematisch den Aufbau einer Partikelstrahlapplikationsvorrichtung 31. In die Partikelstrahlapplikationsvorrichtung 31 tritt ein eintretender Partikelstrahl 33 - üblicherweise ein Partikelstrahl mit einem punktförmigen Querschnittsprofil - ein. Die Partikelstrahlapplikationsvorrichtung 31 umfasst eine Strahlformungsvorrichtung 35, mit der der eintretender Partikelstrahl 33 derart verändert und geformt wird, dass ein geformter Partikelstrahl 37, der aus der Strahlformungsvorrichtung 35 austritt, ein linienförmiges Querschnittsprofil 39 aufweist.

Das linienförmige Querschnittsprofil 39 kennzeichnet sich dadurch aus, dass die Ausdehnung des Partikelstrahls in einer Dimension wesentlich kleiner ist als die Ausdehnung des Partikelstrahls in einer anderen, hierzu senkrechten Dimension. Beispielsweise kann die Ausdehnung des Partikelstrahls in einer Dimension wenige Millimeter betragen, beispielsweise 1mm, 2mm oder auch 3mm oder etwas mehr, während die Ausdehnung des Partikelstrahls in der anderen Dimension im Zentimeterbereich liegt, d.h. größer als einige cm Zentimeter ist, beispielsweise größer als 10 cm, insbesondere größer als 20 cm und höchst insbesondere größer als 30 cm, bis hin zu 50 cm oder mehr.

Der geformte Partikelstrahl 37 wird anschließend auf ein zu bestrahlendes Zielvolumen 41 gelenkt. Um das gesamte Zielvolumen 41 bestrahlend zu können, wird der geformte Partikelstrahl 37 mit dem linienförmigen Querschnittsprofil 39 über das Zielvolumen 41 gescannt. Dies bedeutet, dass der geformte Partikelstrahl 37 seine Energie sukzessive an nebeneinander liegenden Orten im Zielvolumen 41 abgibt. Hierzu weist die Partikelstrahlapplikationsvorrichtung 31 eine Steuervorrichtung 43 zum Ändern der Relativposition zwischen dem Partikelstrahl 37 und dem Zielvolumen 41 auf. Diese Änderung der Relativposition ist in Fig. 2 durch einen Doppelpfeil 45 symbolisiert.

Anhand der nun folgenden Fig. 3 bis Fig. 6 werden verschiedene Partikelstrahlapplikationsvorrichtungen 31 erläutert, in denen die Strahlformungsvorrichtung 35 und/oder die Steuervorrichtung 43 zum Ändern der Relativposition zwischen dem Partikelstrahl 37 und dem Zielvolumen 41 jeweils unterschiedlich ausgebildet sind.

Fig. 3 zeigt eine Strahlformungsvorrichtung 35, die einen Kollimator 47 mit einer linienförmigen Öffnung 49 umfasst. Im Strahlverlauf vor diesem Kollimator 47 ist eine Streuvorrichtung 51 angeordnet. Ein eintretender Partikelstrahl 33, der auf die Streuvorrichtung 51 trifft, wird kegelförmig aufgeweitet. Indem der kegelförmig aufgeweitete Partikelstrahl 53 durch die linienförmige Öffnung 49 des Kollimators 47 hindurch tritt, erhält der Partikelstrahl ein linienförmiges Querschnittsprofil 39.

Der hier gezeigte Kollimator 47 weist dabei eine Einstellvorrichtung 55 zum Ändern der Geometrie der linienförmigen Öffnung 49 des Kollimators 47 auf. Hier gezeigt ist eine Einstellvorrichtung 55 mit einer Vielzahl von Zähnen, die in die linienförmige Öffnung 49 des Kollimators 47 geschoben werden können. Durch diese Zähne kann beispielsweise die Länge der linienförmigen Öffnung 49 des Kollimators 47 begrenzt und variabel eingestellt werden. Durch diese Zähne kann aber auch die linienförmige Öffnung 49 des Kollimators 47 in der Mitte unterbrochen werden, so dass der geformte Partikelstrahl 37 ein unterbrochenes linienförmiges Querschnittsprofil 39 aufweist. Hierdurch ist es möglich, auf einfache Weise auch Zielvolumina "mit einem Loch", d.h. also Zielvolumina mit einer toroidalen Form zu bestrahlen.

Insbesondere wenn der Partikelstrahl 37 mit dem linienförmigen Querschnittsprofil 39 über ein Zielvolumen 41 gescannt wird, kann mit der Einstellvorrichtung 55 die Geometrie der linienförmigen Öffnung 49 des Kollimators 47 geändert werden. Die Änderungen können beispielsweise durch einen Bestrahlungsplan, indem die Geometrie des Zielvolumens 41 hinterlegt ist, gesteuert und umgesetzt werden.

In dem in Fig. 3 gezeigten Beispiel erfolgt das Scannen dadurch, dass die linienförmige Öffnung 49 des Kollimators 47 senkrecht zum Strahlverlauf des Partikelstrahls verschiebbar angeordnet ist. Dies kann beispielsweise dadurch geschehen, dass der gesamte Kollimator 47 senkrecht zum Strahlverlauf des Partikelstrahls verschiebbar gelagert ist. Die Position der linienförmigen Öffnung 49 des Kollimators 47 im Strahlverlauf wird dabei durch die Steuervorrichtung 43 zum Ändern der Relativposition gesteuert. Die Steuervorrichtung 43 kann dabei auch gleichzeitig die Steuerung der Einstellvorrichtung 55 zum Ändern der Geometrie der linienförmigen Öffnung 49 übernehmen.

Fig. 4 zeigt eine ähnliche Strahlformungsvorrichtung 35 wie in Fig. 3. Der Übersichtlichkeit halber ist die Einstellvorrichtung zum Ändern der Geometrie der linienförmigen Öffnung 49 nicht gezeigt. Im Gegensatz zu Fig. 3 wird bei der in Fig. 4 gezeigten Partikelstrahlapplikationsvorrichtung 31 ein Scannen des geformten Partikelstrahls 37 über das Zielvolumen 41 dadurch ermöglicht, dass das Zielvolumen 41 über eine Positioniervorrichtung 57 während eines Bestrahlungsvorgangs beweglich gelagert ist. Durch ein beispielsweise kontinuierliches Bewegen des Zielvolumens 41 scannt der geformte Partikelstrahl 37 mit dem linienförmigen Querschnittsprofil 39 über das Zielvolumen 41.

Hierzu ist die Steuervorrichtung 43 zum Ändern der Relativposition zwischen Partikelstrahl und Zielvolumen derart ausgebildet, dass mit ihr ein Steuersignal für die Positioniervorrichtung 57 für das Zielvolumen 41 ausgegeben werden kann. Die Positioniervorrichtung 57 kann insbesondere eine Komponente der Partikelstrahlapplikationsvorrichtung 31 sein.

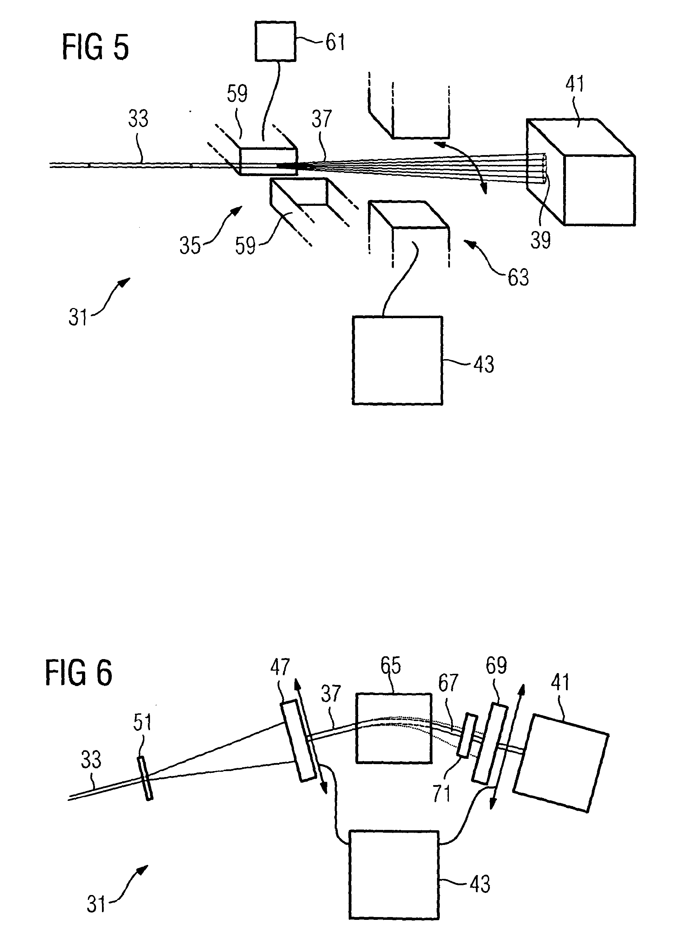

Fig. 5 zeigt eine erfindungsgemäße Ausführungsvariante der Partikelstrahlapplikationsvorrichtung 31.

Die Strahlformungsvorrichtung 35 umfasst dabei ein Magnetsystem 59, mit dem ein oszillierendes Magnetfeld erzeugt werden kann. Ein in dieses Magnetsystem 59 eintretender Partikelstrahl 33 mit einem punktförmigen Querschnittsprofil wird in einer Richtung, die im Wesentlichen senkrecht zur Strahlverlaufsrichtung verläuft, schnell oszillierend abgelenkt. Durch die schnelle Oszillation tritt aus dem Magnetsystem 59 ein geformter Partikelstrahl 37 aus, der ein im Wesentlichen linienförmiges Querschnittsprofil 39 aufweist.

Das Magnetsystem 59 wird durch eine Magnetsystemsteuervorrichtung 61 gesteuert, mit der beispielsweise ein Offset und/oder eine Amplitude und/oder eine Frequenz des oszillierende Magnetfeldes eingestellt werden kann. Insbesondere sind diese Parameter während eines Bestrahlungsvorgangs variabel einstellbar. Hierdurch kann erreicht werden, dass der geformte Partikelstrahl 37 während eines Scan-Vorgangs genau an die Form des Zielvolumens 41 angepasst werden kann.

Weiterhin weist die Partikelstrahlapplikationsvorrichtung 31 eine elektromagnetische Ablenkvorrichtung 63 auf. Diese Ablenkvorrichtung 63 ist im Strahlverlauf hinter dem Magnetsystem 59 angeordnet. Ein geformter Partikelstrahl 37, der durch diese Ablenkvorrichtung 63 hindurch tritt, wird durch das von der Ablenkvorrichtung 63 erzeugte Magnetfeld aus seiner NullPosition heraus abgelenkt. Durch Ändern des Magnetfelds der Ablenkvorrichtung 63 kann der geformte Partikelstrahl 37 über das Zielvolumen 41 gescannt werden. Die elektromagnetische Ablenkvorrichtung 63 wird durch die Steuervorrichtung 43 zum Ändern der Relativposition gesteuert.

Fig. 6 zeigt eine Draufsicht auf eine vorteilhafte Weiterbildung der Partikelstrahlapplikationsvorrichtung 31, mit der ein durch die Partikelstrahlapplikationsvorrichtung 31 geführter Partikelstrahl von Streustrahlung gereinigt werden kann.

Insbesondere, wenn der Partikelstrahl mit dem linienförmigen Querschnittsprofil durch eine Streuvorrichtung 51 zur Aufweitung eines eintretenden Partikelstrahls 33 und einen Kollimator 47 mit einer linienförmigen Öffnung 49 erzeugt wird, entsteht Streustrahlung. Diese Streustrahlung "verunreinigt" den Partikelstrahl, der nun Teilchen aufweist, die ein nicht gewünschtes Masse-zu-Ladungs-Verhältnis aufweisen oder deren Energie von einer gewünschten Energie abweicht. Der geformte Partikelstrahl 37 wird nun durch einen Dipolmagneten 65 geführt und durch diesen Dipolmagneten 65 abgelenkt. Die nicht gewünschten Teilchen des geformten Partikelstrahls 37 erfahren dabei eine unterschiedliche Ablenkung, verglichen mit dem gewünschten Partikeln des Partikelstrahls. Ein aus dem Dipolmagneten 65 austretender Partikelstrahl 67 besteht daher aus Partikeln, deren Energie bzw. deren Masse-zu-Ladungs-Verhältnis deutlich einheitlicher ist als der in den Dipolmagneten 65 eintretende geformte Partikelstrahl 37, der mit Streustrahlung verunreinigt ist.

Nach dem Dipolmagneten 65 kann ein zweiter Kollimator 69 mit einer linienförmigen Öffnung angeordnet sein, durch den der austretende Partikelstrahl 67 hindurch tritt. Der zweite Kollimator 69 ist dabei derart positioniert, dass Partikel mit der gewünschten Energie bzw. mit dem gewünschten Masse-zu-Ladungs-Verhältnis durch die linienförmige Öffnung des zweiten Kollimators 69 hindurch treten. Hierdurch erfolgt eine weitere Filterung von Partikeln, die eine nicht gewünschte Energie bzw. ein nicht gewünschtes Masse-zu-Ladungs-Verhältnis aufweisen. Derartige Partikel sind nämlich durch den Dipolmagneten 65 unterschiedlich abgelenkt worden, so dass sie nicht durch die linienförmige Öffnung des zweiten Kollimators 69 treten.

Insbesondere, wenn die linienförmige Öffnung des ersten Kollimators 47 im Partikelstrahl z.B. während eines Bestrahlungsvorgangs bewegt wird, erfolgt eine Bewegung der linienförmigen Öffnung des zweiten Kollimators 69 im Strahlverlauf in entsprechender Weise. Die abgestimmte Bewegung der beiden Kollimatoren zueinander kann durch die Steuervorrichtung 43 zum Ändern der Relativposition gesteuert werden.

In Fig. 6 ist zusätzlich eine weitere optionale Komponente der Partikelstrahlapplikationsvorrichtung gezeigt. Im Strahlverlauf ist eine Tiefenmodulationsvorrichtung 71 zur Beeinflussung der Partikelstrahlenergie angeordnet. Mit einer derartigen Tiefenmodulationsvorrichtung 71 kann die Energie der Partikel des Partikelstrahls und damit die Eindringtiefe des Partikelstrahls in das Zielvolumen moduliert werden. Zur Beeinflussung der Partikelstrahlenergie können beispielsweise ein so genanntes Modulator-Wheel oder ein so genannter Ridge-Filter eingesetzt werden. Alternativ ist es möglich, die Energie der Partikel des Partikelstrahls z.B. durch Steuerung der Beschleuniger, mit dem die Partikel beschleunigt werden, einzustellen. In Fig. 6 ist die Tiefenmodulationsvorrichtung 71 nach dem Dipolmagneten 65 angeordnet und vor dem zweiten Kollimator 69. Es sind aber auch andere Anordnungen der Tiefenmodulationsvorrichtung denkbar, beispielsweise nach dem zweiten Kollimator 69 oder auch an anderer Stelle.

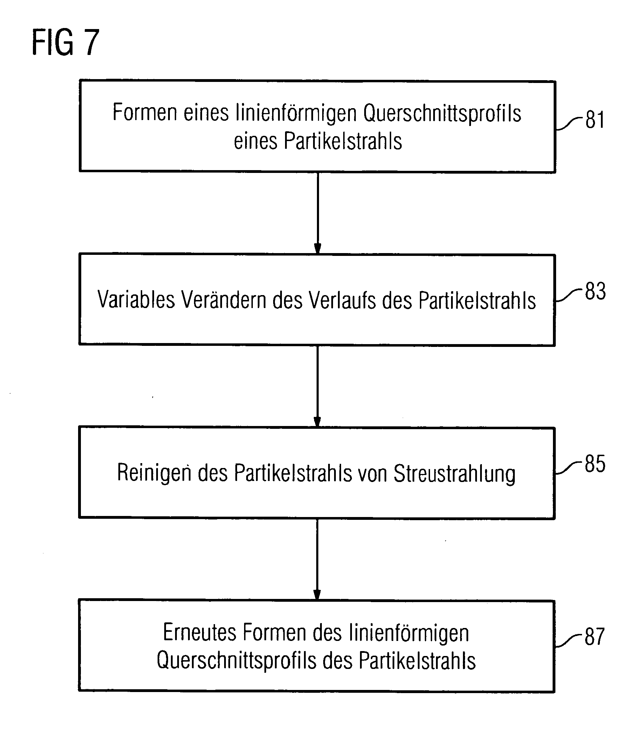

Fig. 7 zeigt einen schematischen Überblick über die Verfahrensschritte einer Ausführungsform des Verfahrens zur Führung eines Partikelstrahls.

In einem ersten Schritt 81 wird das Querschnittsprofil eines Partikelstrahls derart geformt, dass der Partikelstrahl ein linienförmiges Querschnittsprofil aufweist. Hierzu kann der Partikelstrahl zuerst aufgefächert und anschließend sein Querschnittsprofil geformt werden. In einem zweiten Schritt 83 wird der Verlauf des Partikelstrahls in einer Richtung variabel geändert, die senkrecht zur Strahlverlaufsrichtung des Partikelstrahls und insbesondere senkrecht zum linienförmigen Verlauf des linienförmigen Querschnittsprofils verläuft. Durch das variable Ändern wird der Partikelstrahl derart geführt, dass der Partikelstrahl in unterschiedliche Richtungen gelenkt wird, wodurch beispielsweise ein Scan-Vorgang ermöglicht wird.

Nach Formen und Ändern des Verlaufs des Partikelstrahls kann der Partikelstrahl in einem optionalen, dritten Schritt 85 von Streustrahlung gereinigt werden, indem der Partikelstrahl durch ein weiteres, zweites Magnetfeld geführt und in seiner Richtung abgelenkt wird. Insbesondere kann der Partikelstrahl nach der Reinigung von Streustrahlung ein weiteres Mal in einem optionalen, vierten Schritt 87 in seinem Querschnittsprofil insbesondere durch einen Kollimator geformt werden. Ein auf diese Weise geformter bzw. geführter Partikelstrahl kann für verschiedene Zwecke eingesetzt werden, beispielsweise für Forschungszwecke, zur Bestrahlung von Zielvolumina, etc.

## Patentansprüche

1. Partikelstrahlapplikationsvorrichtung, mit der ein in die Partikelstrahlapplikationsvorrichtung eintretender Partikelstrahl auf ein Zielvolumen zur Bestrahlung des Zielvolumens richtbar ist, umfassend:
- eine in einem Strahlengang des Partikelstrahls anordenbare Strahlformungsvorrichtung zur Formung eines Querschnittsprofils des Partikelstrahls, wobei die Strahlformungsvorrichtung ein Magnetsystem umfasst und derart ausgebildet ist, dass der Partikelstrahl nach der Formung ein linienförmiges Querschnittsprofil aufweist,
- eine Steuervorrichtung zum Ändern der Relativposition des Partikelstrahls und des Zielvolumen während eines Bestrahlungsvorgangs, wodurch der Partikelstrahl mit dem linienförmigen Querschnittsprofil über das zu bestrahlende Zielvolumen scanbar ist,
**dadurch gekennzeichnet, dass**
mit dem Magnetfeld der Stahlformungsvorrichtung ein oszillierendes Magnetfeld erzeugbar ist, wodurch ein in das Magnetsystem eintretender Partikelstrahl, der ein punktförmiges Querschnittsprofil aufweist, in einer Richtung, die im Wesentlichen senkrecht zur Strahlverlaufsrichtung verläuft, derart oszillierend ablenkbar ist, dass durch eine Oszillation ein linienförmiges Querschnittsprofil formbar ist, wobei die Strahlformungsvorrichtung zusätzlich eine Magnetsystemsteuervorrichtung umfasst, mit der ein Offset und/oder eine Amplitude und/oder eine Frequenz des oszillierenden Magnetfeldes variabel einstellbar ist.

2. Partikelstrahlapplikationsvorrichtung nach Anspruch 1, wobei mit der Magnetsystemsteuervorrichtung der Offset und/oder die Amplitude und/oder die Frequenz des oszillierenden Magnetfeldes während eines Bestrahlungsvorganges variabel einstellbar ist.

3. Partikelstrahlapplikationsvorrichtung nach einem der Ansprüche 1 oder 2, wobei
- die Partikelstrahlapplikationsvorrichtung eine elektromagnetische Ablenkvorrichtung aufweist, die insbesondere im Strahlverlauf hinter der Strahlformungsvorrichtung angeordnet ist, und wobei
- die elektromagnetische Ablenkvorrichtung durch die Steuervorrichtung zum Ändern der Relativposition zwischen dem Partikelstrahl und dem zu bestrahlenden Zielvolumen derart steuerbar ist, dass der Partikelstrahl mit dem linienförmigen Querschnittsprofil ablenkbar und hierdurch über das Zielvolumen scanbar ist.

4. Partikelstrahlapplikationsvorrichtung nach einem der Ansprüche 1 bis 3, wobei
- die Steuervorrichtung zum Ändern der Relativposition zwischen dem Partikelstrahl und dem zu bestrahlenden Zielvolumen zur Abgabe eines Steuersignals für eine Positioniervorrichtung für das Zielvolumen ausgebildet ist, so dass während eines Bestrahlungsvorgangs das Zielvolumen mit der Positioniervorrichtung bewegbar ist, wodurch der Partikelstrahl mit dem linienförmigen Querschnittsprofil über das Zielvolumen scanbar ist.

5. Partikelstrahlapplikationsvorrichtung nach einem der Ansprüche 1 bis 4, wobei die Partikelstrahlapplikationsvorrichtung zusätzlich aufweist:
- zumindest einen im Strahlverlauf hinter der Strahlformungsvorrichtung angeordneten Dipolmagneten zur Reinigung des Partikelstrahls von Streustrahlung.

6. Partikelstrahlapplikationsvorrichtung nach Anspruch 5, wobei die Partikelstrahlapplikationsvorrichtung zusätzlich aufweist:
- einen im Strahlverlauf hinter dem Dipolmagneten angeordneten Kollimator mit einer linienförmigen Öffnung.

7. Partikelstrahlapplikationsvorrichtung nach Anspruch 6, wobei der Kollimator derart ausgebildet ist, dass eine Position der linienförmigen Öffnung des Kollimators relativ zum Strahlverlauf veränderbar ist.

8. Partikelstrahlapplikationsvorrichtung nach einem der Ansprüche 1 bis 7, wobei die Partikelstrahlapplikationsvorrichtung zusätzlich aufweist:
- eine Tiefenmodulationsvorrichtung zur Modulation der Eindringtiefe des Partikelstrahls.

9. Bestrahlungsvorrichtung, umfassend:
- zumindest eine Partikelstrahlapplikationsvorrichtung nach einem der Ansprüche 1 bis 8,
- zumindest eine Quelle zur Erzeugung von Partikeln, und
- zumindest eine vor der Partikelstrahlapplikationsvorrichtung angeordnete Beschleunigungsvorrichtung zur Beschleunigung der Partikel und zur Erzeugung des Partikelstrahls aus den beschleunigten Partikeln.

10. Bestrahlungsvorrichtung nach Anspruch 9, wobei bei einer Bestrahlung eines bewegten Zielvolumens die Relativposition zwischen dem Zielvolumen und dem Partikelstrahl derart einstellbar ist, dass eine Hauptbewegungsrichtung des Zielvolumens parallel zum linienförmigen Querschnittsprofil des Partikelstrahls verläuft.

11. Verfahren zur Führung eines Partikelstrahls, wobei Verfahren zur therapeutischen Bestrahlung eines menschlichen oder tierischen Körpers ausgeschlossen sind, aufweisend folgende Schritte:
- Formen eines Querschnittsprofils eines Partikelstrahls durch ein Magnetfeld derart, dass der Partikelstrahl ein linienförmiges Querschnittsprofil aufweist,
- Ändern eines Verlaufs des Partikelstrahls in einer Richtung, die senkrecht zur Strahlverlaufsrichtung des Partikelstrahls und insbesondere senkrecht zum linienförmigen Querschnittsprofil verläuft,
**dadurch gekennzeichnet, dass**
bei dem Formen des Querschnittsprofils des Partikelstrahls ein oszillierendes Magnetfeld auf einen Partikelstrahl mit einem punktförmigen Querschnittsprofil eingestrahlt wird, wodurch der Partikelstrahl mit dem punktförmigen Querschnittsprofil in einer Richtung, die im Wesentlichen senkrecht zur Strahlverlaufsrichtung verläuft, oszillierend abgelenkt wird, so dass aus dem Partikelstrahl mit dem punktförmigen Querschnittsprofil der Partikelstrahl mit dem linienförmigen Querschnittsprofil erzeugt wird, wobei
das oszillierende Magnetfeld in seinem Offset und/oder in seiner Amplitude und/oder in seiner Frequenz variabel einstellbar ist.

12. Verfahren nach Anspruch 11, wobei das oszillierende Magnetfeld in seinem Offset und/oder in seiner Amplitude und/oder in seiner Frequenz während eines Bestrahlungsvorganges variabel einstellbar ist.

13. Verfahren nach einem der Ansprüche 11 oder 12, wobei
das Ändern des Verlaufs des Partikelstrahls **dadurch** erfolgt, dass zumindest ein Magnetfeld senkrecht zur Verlaufsrichtung des Partikelstrahls eingestrahlt wird.

14. Verfahren nach einem der Ansprüche 11 bis 13, wobei
der Partikelstrahl von Streustrahlung gereinigt wird, indem der Partikelstrahl durch ein zweites Magnetfeld abgelenkt wird.

15. Verfahren nach Anspruch 14, wobei
der Partikelstrahl nach der Reinigung von Streustrahlung ein weiteres Mal in seinem Querschnittsprofil insbesondere durch einen Kollimator geformt wird.

## Claims

1. Particle beam application device with which a particle beam entering the particle beam application device can be directed at a target volume for irradiating said target volume, comprising:
- a beam shaping device which can be disposed in a beam path of the particle beam for shaping a cross-sectional profile of the particle beam, wherein the beam shaping device comprises a magnetic system and is embodied such that, after shaping, the particle beam has a linear cross-sectional profile,
- a controller for varying the relative position of the particle beam and target volume during an irradiation run, by means of which the particle beam with the linear cross-sectional profile can be scanned over the target volume to be irradiated,
**characterised in that** with the magnetic field of the beam shaping device an oscillating magnetic field can be generated by means of which a particle beam with a point-like cross-sectional profile entering the magnetic system can be deflected in an oscillating manner in a direction running essentially perpendicular to the beam direction such that a linear cross-sectional profile can be formed by oscillation, wherein the beam shaping device additionally has a magnetic system controller with which an offset and/or an amplitude and/or a frequency of the oscillating magnetic field can be variably adjusted.

2. Particle beam application device according to claim 1, wherein by means of the magnetic system controller an offset and/or an amplitude and/or a frequency of the oscillating magnetic field can be variably adjusted during an irradiation run.

3. Particle beam application device according to one of claims 1 and 2, wherein
- the particle beam application device has an electromagnetic deflecting device which is disposed in particular in the beam trajectory downstream of the beam shaping device, and wherein
- the electromagnetic deflecting device can be controlled by the controller for varying the relative position between the particle beam and the target volume to be irradiated such that the particle beam with the linear cross-sectional profile can be deflected and thereby scanned over the target volume.

4. Particle beam application device according to one of claims 1 to 3, wherein
- the controller for varying the relative position between the particle beam and the target volume to be irradiated is embodied to produce a control signal for a positioning device for the target volume so that the target volume can be moved using the positioning device during an irradiation run, thereby enabling the particle beam with the linear cross-sectional profile to be scanned over the target volume.

5. Particle beam application device according to one of claims 1 to 4, wherein the particle beam application device additionally has:
- at least one dipole magnet disposed downstream of the beam shaping device in the beam trajectory for de-scattering the particle beam.

6. Particle beam application device according to claim 5,
wherein the particle beam application device additionally has:
- a collimator with a linear aperture disposed downstream of the dipole magnet in the beam trajectory.

7. Particle beam application device according to claim 6, wherein the collimator is embodied such that a position of the linear aperture of the collimator can be varied relative to the beam trajectory.

8. Particle beam application device according to one of claims 1 to 7, wherein the particle beam application device additionally has:
- a depth modulation device for modulating the penetration depth of the particle beam.

9. Irradiation device, comprising:
- at least one particle beam application device according to one of claims 1 to 8,
- at least one source for generating particles, and
- at least one acceleration device disposed upstream of the particle beam application device for accelerating the particles and producing the particle beam from the accelerated particles.

10. Irradiation device according to claim 9, wherein to irradiate a moving target volume, the relative position between the target volume and the particle beam can be adjusted such that a primary movement direction of the target volume runs parallel to the linear cross-sectional profile of the particle beam.

11. Method for guiding a particle beam, wherein methods for the therapeutic irradiation of a human or animal body are excluded, having the following steps:
- shaping a cross-sectional profile of a particle beam by a magnetic field such that the particle beam has a linear cross-sectional profile,
- changing a trajectory of the particle beam in a direction running perpendicular to the beam direction of the particle beam and in particular perpendicular to the linear cross-sectional profile.
**characterised in that**,
to shape the cross-sectional profile of the particle beam, an oscillating magnetic field is applied to a particle beam with a point-like cross-sectional profile, causing the particle beam with the point-like cross-sectional profile to be deflected in an oscillating manner in a direction running essentially perpendicular to the beam direction, so that the particle beam with the linear cross-sectional profile is produced from the particle beam with the point-like cross-sectional profile, wherein
the offset and/or amplitude and/or frequency of the oscillating magnetic field can be variably adjusted.

12. Method according to claim 11, wherein the offset and/or amplitude and/or frequency of the oscillating magnetic field can be variably adjusted during an irradiation run.

13. Method according to one of claims 11 or 12,
wherein the trajectory of the particle beam is changed by applying at least one magnetic field perpendicular to the direction of the particle beam.

14. Method according to one of claims 11 to 13, wherein
the particle beam is de-scattered by its deflection by a second magnetic field.

15. Method according to claim 14,
wherein after being de-scattered, the cross-sectional profile of the particle beam is reshaped, in particular by a collimator.

## Revendications

1. Dispositif d'application de faisceau de particules, permettant de diriger un faisceau de particules entrant dans le dispositif d'application de faisceau de particules sur un volume cible afin d'irradier le volume cible, comprenant :
- un dispositif de conformation de faisceau apte à être disposé sur une trajectoire du faisceau de particules pour former un profil de section transversale du faisceau de particules, ledit dispositif de conformation de faisceau comprenant un système magnétique et étant configuré de telle façon que le faisceau de particules, après la conformation, présente un profil de section transversale linéaire,
- un dispositif de commande pour modifier la position relative du faisceau de particules et du volume cible pendant un processus d'irradiation, permettant ainsi de scanner le faisceau de particules au profil de section transversale linéaire par-dessus le volume cible à irradier,
**caractérisé en ce que** le champ magnétique du dispositif de conformation de faisceau permet de créer un champ magnétique oscillant, grâce auquel un faisceau de particules entrant dans le système magnétique, et qui présente un profil de section transversale ponctuel, peut être dévié de manière oscillante selon une direction s'étendant essentiellement perpendiculairement à la direction du trajet du faisceau de telle façon que, par une oscillation, un profil de section transversale linéaire peut être formé, le dispositif de conformation de faisceau comprenant en outre un dispositif de commande de système magnétique, permettant d'ajuster d'une façon variable un offset et/ou une amplitude et/ou une fréquence du champ magnétique oscillant.

2. Dispositif d'application de faisceau de particules selon la revendication 1, ledit dispositif de commande de système magnétique permettant d'ajuster d'une façon variable l'offset et/ou l'amplitude et/ou la fréquence du champ magnétique oscillant pendant un processus d'irradiation.

3. Dispositif d'application de faisceau de particules selon l'une des revendications 1 ou 2,
- ledit dispositif d'application de faisceau de particules comprenant un dispositif de déviation électromagnétique, disposé notamment sur le trajet du faisceau en aval du dispositif de conformation de faisceau, et
- ledit dispositif de déviation électromagnétique pouvant être commandé par le dispositif de commande apte à modifier la position relative entre le faisceau de particules et le volume cible à irradier, de telle façon que le faisceau de particules au profil de section transversale linéaire peut être dévié et ainsi être scanné par-dessus le volume cible.

4. Dispositif d'application de faisceau de particules selon l'une des revendications 1 à 3,
- ledit dispositif de commande apte à modifier la position relative entre le faisceau de particules et le volume cible à irradier étant configuré pour délivrer un signal de commande pour un dispositif de positionnement du volume cible de sorte que le volume cible peut être déplacé par le dispositif de positionnement pendant un processus d'irradiation, permettant ainsi de scanner le faisceau de particules au profil de section transversale linéaire par-dessus le volume cible.

5. Dispositif d'application de faisceau de particules selon l'une des revendications 1 à 3, ledit dispositif d'application de faisceau de particules comprenant en outre :
- au moins un aimant dipolaire disposé sur le trajet du faisceau en aval du dispositif de conformation de faisceau pour débarrasser le faisceau de particules de rayonnement diffus.

6. Dispositif d'application de faisceau de particules selon la revendication 5, ledit dispositif d'application de faisceau de particules comprenant en outre :
- un collimateur pourvu d'une ouverture linéaire, disposé sur le trajet du faisceau en aval de l'aimant dipolaire.

7. Dispositif d'application de faisceau de particules selon la revendication 6, ledit collimateur étant configuré de telle façon qu'une position de l'ouverture linéaire du collimateur par rapport au trajet du faisceau peut être modifiée.

8. Dispositif d'application de faisceau de particules selon l'une des revendications 1 à 7, ledit dispositif d'application de faisceau de particules comprenant en outre :
- un dispositif de modulation de profondeur pour moduler la profondeur de pénétration du faisceau de particules.

9. Dispositif d'irradiation, comprenant :
- au moins un dispositif d'application de faisceau de particules selon l'une des revendications 1 à 8,
- au moins une source de génération de particules, et
- au moins un dispositif d'accélération disposé en amont du dispositif d'application de faisceau de particules pour accélérer les particules et pour produire le faisceau de particules à partir des particules accélérées.

10. Dispositif d'irradiation selon la revendication 9,
lors d'une irradiation d'un volume cible en mouvement la position relative entre le volume cible et le faisceau de particules pouvant être ajustée de telle façon qu'une direction de mouvement principal du volume cible s'étend parallèlement au profil de section transversale linéaire du faisceau de particules.

11. Procédé de guidage d'un faisceau de particules, les procédés d'irradiation thérapeutique d'un corps humain ou animal étant exclus, comprenant les étapes suivantes :
- former un profil de section transversale d'un faisceau de particules par un champ magnétique de telle façon que le faisceau de particules présente un profil de section transversale linéaire,
- modifier un trajet d'un faisceau de particules selon une direction s'étendant perpendiculairement à la direction du trajet du faisceau de particules, et en particulier perpendiculairement au profil de section transversale linéaire,
**caractérisé en ce que**
lors de formation du profil de section transversale du faisceau de particules un champ magnétique oscillant est appliqué sur un faisceau de particules présentant un profil de section transversale ponctuel, ce qui fait dévier le faisceau de particules au profil de section transversale ponctuel de manière oscillante selon une direction s'étendant essentiellement perpendiculairement à la direction du trajet du faisceau de particules, permettant ainsi de créer à partir du faisceau de particules au profil de section transversale ponctuel le faisceau de particules au profil de section transversale linéaire,
l'offset et/ou l'amplitude et/ou la fréquence du champ magnétique oscillant étant ajustable(s) d'une façon variable.

12. Procédé selon la revendication 11,
l'offset et/ou l'amplitude et/ou la fréquence du champ magnétique oscillant étant ajustable(s) d'une façon variable pendant un processus d'irradiation.

13. Procédé selon l'une des revendications 11 ou 12,
la modification du trajet du faisceau de particules se faisant en appliquant au moins un champ magnétique perpendiculairement à la direction du trajet du faisceau de particules.

14. Procédé selon l'une des revendications 11 à 13,
le faisceau de particules étant débarrassé de rayonnement diffus en faisant dévier le faisceau de particules par un deuxième champ magnétique.

15. Procédé selon la revendication 14,
le profil de section transversale du faisceau de particules, une fois ce dernier débarrassé de rayonnement diffus, est formé une nouvelle fois, en particulier par un collimateur.
